# EUROPEAN PATENT APPLICATION

(11) **EP 3 000 379 A1**
(43) Date of publication of application: **30.03.2016**
(21) Application number: 14801787.4
(22) Date of filing: 09.05.2014
(51) Int. Cl.: A61B 1/00, G02B 23/24

(54) **CURVED SENSOR**

(30) Priority: 22.05.2013 JP 2013108152
(71) Applicant: OLYMPUS CORPORATION, Shibuya-ku Tokyo 151-0072 (JP)
(72) Inventor: KUBOI, Toru, 151-0072 Tokyo (JP); SATO, Eijiro, 151-0072 Tokyo (JP)
(74) Representative: Winter, Brandl, Fürniss, Hübner, Röss, Kaiser, Polte - Partnerschaft mbB
(86) International application number: PCT/JP2014/062477
(87) International publication number: WO 2014/188885

(57) **Abstract**

A rotation suppressing member 211 is fixed to a detection optical fiber 103a of a curved shape sensor. The rotation suppressing member 211 is located within the space defined by ring members 201, guides 204 and CH tube 208 with being in contact with the ring members 201, guides 204 and CH tube 208. In this manner, the rotation suppressing member 211 suppresses rotation of the detection optical fiber 103a.

## Description

### [Technical Field]

The present invention relates to a curved shape sensor for detecting the curved shape of a measured object.

### [Background Art]

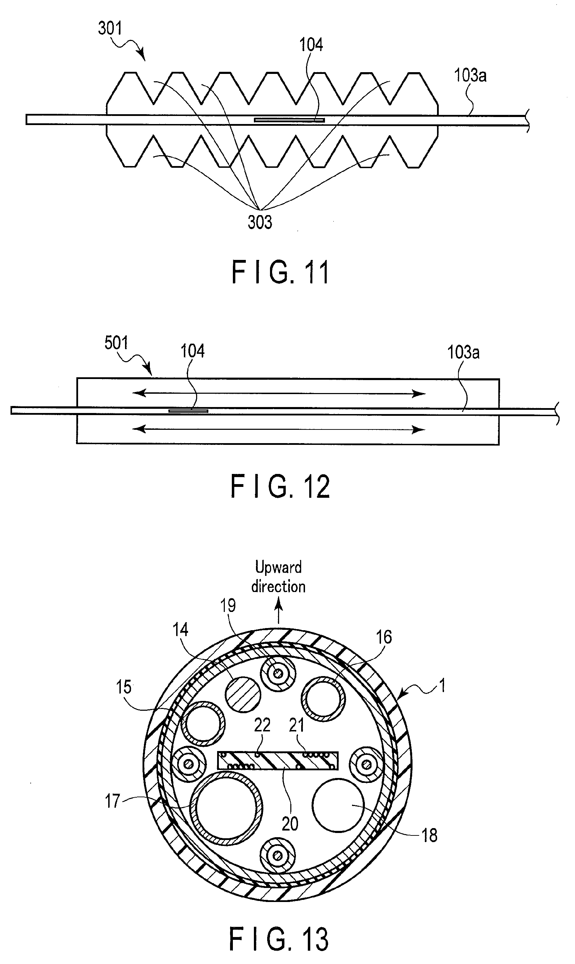

Jpn. Pat. Appln. KOKAI Publication No. 2003-52614 discloses an example of a curved shape sensor. FIG. 13 is a sectional view showing an insertion-section flexible tube provided at an insertion section of an endoscope in which the curved shape sensor is incorporated.

As shown in FIG. 13, a band member 20 is incorporated in the flexible tube 1 of the insertion section of the endoscope. The band member 20 is provided with a curve detector 22 constituted by a curve-detecting optical fiber 21. Since when the insertion-section flexible tube 1 is curved, the band member 20 is also curved in a similar shape, the shape of the insertion-section flexible tube 1, namely, the curved shape of the endoscope, can be detected.

### [Summary of the Invention]

In the insertion-section flexible tube 1 shown in FIG. 13, a video signal transmission cable 14, air/water supply tubes 15 and 16, a treatment tool insertion channel 17, an illumination light guide 18, and a bending-operation wire 19 are located around the band member 20. Of these members, only the treatment tool insertion channel 17 is in contact with the band member 20, and the other members are kept separated from the band member 20, with a space provided in between.

With this structure, when the insertion-section flexible tube 1 is curved, the band member 20, video signal transmission cable 14, air/water supply tubes 15 and 16, treatment tool insertion channel 17, illumination light guide 18, and bending-operation wire 19 are moved, and as a result the band member 20 may be exerted with an external force and twisted.

If the band member 20 is twisted, the curve detector in the band member 20 is inclined in accordance with the twist. As a result, an optical signal obtained from the curve-detecting optical fiber having directional property may vary, and the curvature and the curved direction of the insertion-section flexible tube 1 may not be accurately detected.

The present invention has been made in consideration of these circumstances, and is intended to provide a curved shape sensor preventing the detection optical fiber from being twisted.

The present invention provides a curved shape sensor for detecting the curved shape of a measured object. The curved shape sensor comprises a light source to emit detection light, an optical fiber to guide the detection light, a detection part provided at a portion of the optical fiber, a rotation suppressing member fixed to the optical fiber, and a light detection unit to detect the detection light traveling through the optical fiber. The measured obj ect includes a tubular member that has flexibility so as to be curved at least in one direction, and an incorporation member located inside the tubular member. The detection part changes the characteristics of the detection light passing therethrough in accordance with a change in the curvature of the optical fiber. The rotation suppressing member is located within the space defined by the tubular member and the incorporation member with being in contact with the tubular member and the incorporation member, so as to suppress rotation of the optical fiber.

The present invention provides a curved shape sensor preventing the detection optical fiber from being twisted.

### [Brief Description of Drawings]

FIG. 1 is a schematic diagram illustrating the operating principle underlying a curved shape sensor.
FIG. 2 is a cross section of a detection part of the curved shape sensor.
FIG. 3 is a whole structure diagram of an endoscope system.
FIG. 4 is an axial-direction section of a distal insertion tube of an endoscope in which the curved shape sensor of the first embodiment is incorporated, the section being taken along line A-A of FIG. 5.
FIG. 5 is a radial-direction section of the distal insertion tube of the endoscope in which the curved shape sensor of the first embodiment is incorporated, the section being taken along line B-B of FIG. 4.
FIG. 6 shows an example of the rotation suppressing member depicted in FIG. 5.
FIG. 7 shows another example of the rotation suppressing member depicted in FIG. 5.
FIG. 8 is a radial-direction section of the distal insertion tube of an endoscope in which a curved shape sensor of the second embodiment is incorporated.
FIG. 9 is a radial-direction section of the distal insertion tube of an endoscope in which a curved shape sensor of the third embodiment is incorporated.
FIG. 10 shows an example of the tape member depicted in FIG. 9.
FIG. 11 shows another example of the tape member depicted in FIG. 9.
FIG. 12 shows an example of a tape member of the fourth embodiment.
FIG. 13 is a radial-direction section of the distal insertion tube of an endoscope in which a conventional curved shape sensor is incorporated.

### [Description of Embodiments]

### <First Embodiment>

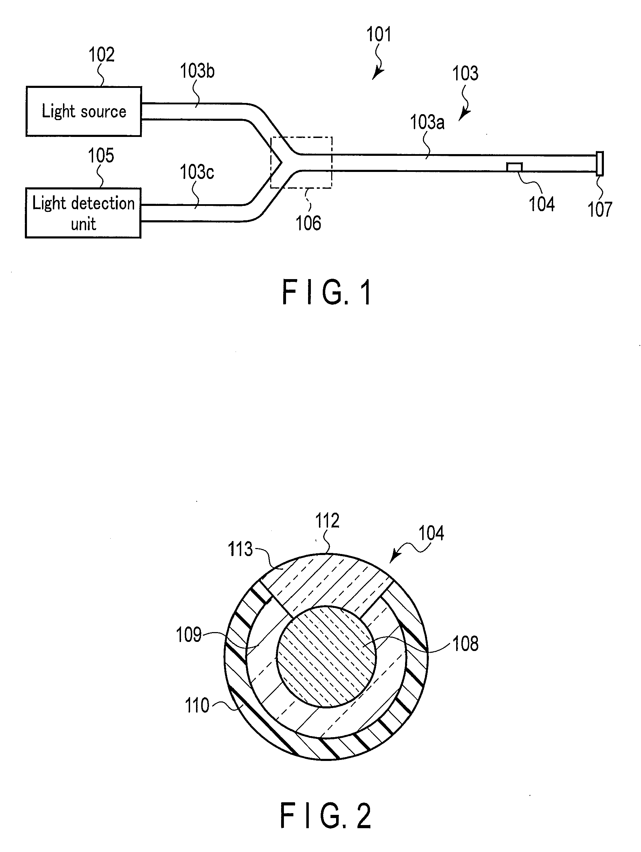

The operating principle underlying a curved shape sensor 101 will be described with reference to the diagrams shown in FIGS. 1 and 2. FIG. 1 is a schematic diagram illustrating the operating principle underlying a curved shape sensor, and FIG. 2 is a cross section of a detection part of the curved shape sensor.

The curved shape sensor 101 comprises a light source 102 to emit detection light, an optical fiber 103 to guide the detection light emitted from the light source, a detection part 104 provided at a portion of the optical fiber 103, and a light detection unit 105 to detect the detection light traveling through the optical fiber. The light source 102 is, for example, a light emitting diode (LED) or a laser source.

The optical fiber 103 is branched in three directions at a coupler (an optical coupler) 106, and constituted by a detection optical fiber 103a, a light-supplying optical fiber 103b, and a light-receiving optical fiber 103c. A reflector 107 to reflect the traveling light is provided at the distal end of the detection optical fiber 103a. As shown in FIG. 2, the optical fiber 103 comprises a core 108 and a clad covering the outer circumference of the core 108, and may include a coating member 110 as an outermost layer.

The coupler 106 is configured to couple two light guide members, namely, the light-supplying optical fiber 103b and the light-receiving optical fiber 103c, with one light guide member, namely, the detection optical fiber 103a. The light-supplying optical fiber 103b is a light introduction path and guides the light emitted from the light source 102 located at an end to the coupler 106. The coupler 106 has the function of guiding most of the light entering from the light-supplying optical fiber 103b to the detection optical fiber 103a and guiding at least a portion of the light reflected by the reflector 107 to the light-receiving optical fiber 103c.

In the curved shape sensor 101 according to the present embodiment, the detection optical fiber 103a is integrally attached to, and extended along an elongated flexible bending structure, which is a measured object, such as the distal insertion tube of an endoscope, so that the curved state and the curved direction of the flexible bending structure are detected. When the curved shape sensor 101 is attached to a measured object, the curvable portion of the measured object is positioned with reference to the detection part 104 of the curved shape sensor 101, so that the detection part 104 is located at the proper position of the measured object. The detection optical fiber 103a moves in accordance with the flexible movement of the measured object, and permits the light entering from the light-supplying optical fiber 103b to be reflected by the reflector 107 located at the distal end. The light is therefore allowed to travel back and forth through the detection optical fiber 103a. To be more specific, the detection optical fiber 103a guides the light traveling from the light-supplying optical fiber 103b and through the coupler 106 to the reflector 107, and returns the light reflected by the reflector 107 to the coupler 106.

The light-receiving optical fiber 103c is a light guide path and guides the light reflected by the reflector 107 and branched at the coupler 106 to a light detection unit 105. The detecting optical fiber 103a includes at least one detection part 104. The detection part changes the characteristics of the detection light passing therethrough in accordance with a change in the curvature of the optical fiber 103a.

As shown in FIG. 2, the detection part 104 includes an open section 112 formed by removing part of the clad 109 from the outer circumference of the detection optical fiber 103 to expose the core 108, and an optical characteristic conversion member 113 located in the open section 112. The open section 112 does not have to be formed to expose the core 108. The only requirement of the open section 112 is that the light traveling through the shape-detecting fiber 103a reaches the open section 112.

The optical characteristic conversion member 113 has a function of converting the optical characteristics of guided light. The optical characteristic conversion member 113 is, for example, a light attenuation member or a wavelength conversion member. For example, the light attenuation member is a light absorber, and the wavelength conversion member is a phosphor member. In the present embodiment, the optical characteristic conversion member 113 is a light attenuation member.

The light emitted from the light source 102 is guided by the light-supplying optical fiber 103b, coupler 106 and detection optical fiber 103a, and is then reflected by the reflector 107. The light reflected by the reflector 107 is branched by the coupler 106 as detection light, is guided through the light-receiving optical fiber 103c, and then reaches the light detection unit 105. The light detection unit 105 photoelectically converts the received detection light and outputs an electric signal representing an amount of light.

The curved shape sensor 101 undergoes an optical loss where the light guided through the optical fiber 103 enters the optical characteristic conversion member 113. The amount of this optical loss varies in accordance with the direction of the curve or deflection of the light-receiving optical fiber 103c and the amount of curve.

Even when the detection optical fiber 103a is straight, a certain amount of light is lost in the optical characteristic conversion member 113, and the amount of loss is dependent on the width of the open section 112. Let us assume that this light loss is used as a reference amount. If the optical characteristic conversion member 113 is located outward with respect to the curved direction of the detection optical fiber 103a, the amount of light that is lost is larger than the reference amount. Conversely, if the optical characteristic conversion member 113 is located inward with respect to the curved direction of the detection optical fiber 103a, the amount of light that is lost is smaller than the reference amount.

Changes in the amount of light loss are reflected in the amount of detection light received by the light detection unit 105. That is, the changes in the amount of light loss are reflected in the output signal of the light detection unit 105. Therefore, in accordance with the output signal of the light detection unit 105, the curved direction and amount (angle) of a measured object can be detected at the detection part of the curved shape sensor 101, i.e., at the position of the optical characteristic conversion member 113.

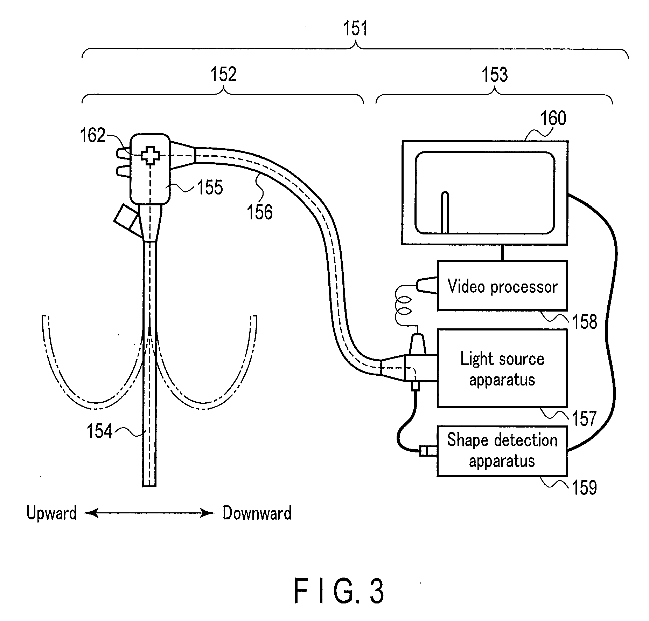

FIG. 3 shows a whole structure diagram of an endoscope system. An endoscope 151 comprises an insertion section 152 and a main body section 153. The insertion section 152 comprises a distal insertion tube 154, a proximal operation section 155, and a connection section 156. The distal insertion tube 154 can be curved in at least one direction (upward or downward) to have a desired curvature by operating a dial 162 on the proximal operation section 155. The proximal operation section 155 is electrically connected to a light source apparatus 157 of the main body section 153 through the connection section 156, and an image sensor, an illumination unit, etc. incorporated in the distal insertion tube 154 can be controlled by the main body section 153.

The main body section 153 comprises a light source apparatus 157, a video processor 158, a shape detection apparatus 159, and a monitor 160, and these structural elements are electrically connected to each other so that signals can be controlled properly.

The endoscope 151 incorporates the curved shape sensor 101 described above, and the detection optical fiber 103a is located in the interior of the distal insertion tube 154. The shape detection apparatus 159 detects the curved direction and amount (angle) of the distal insertion tube 154 based on an output signal of the curved shape sensor 101.

### (Structure)

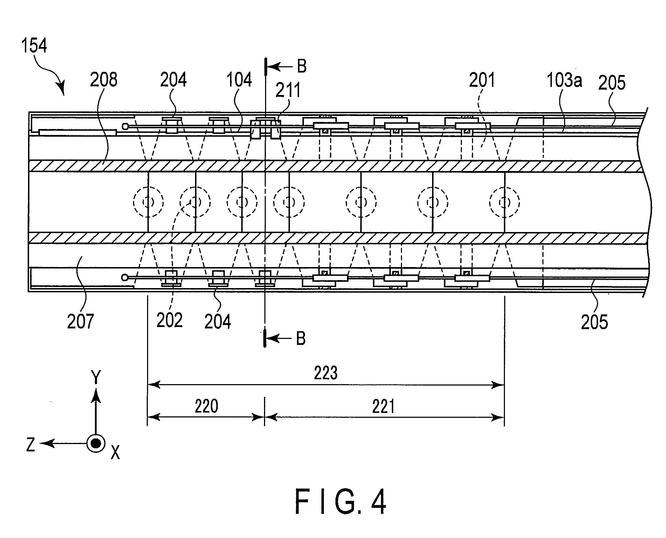

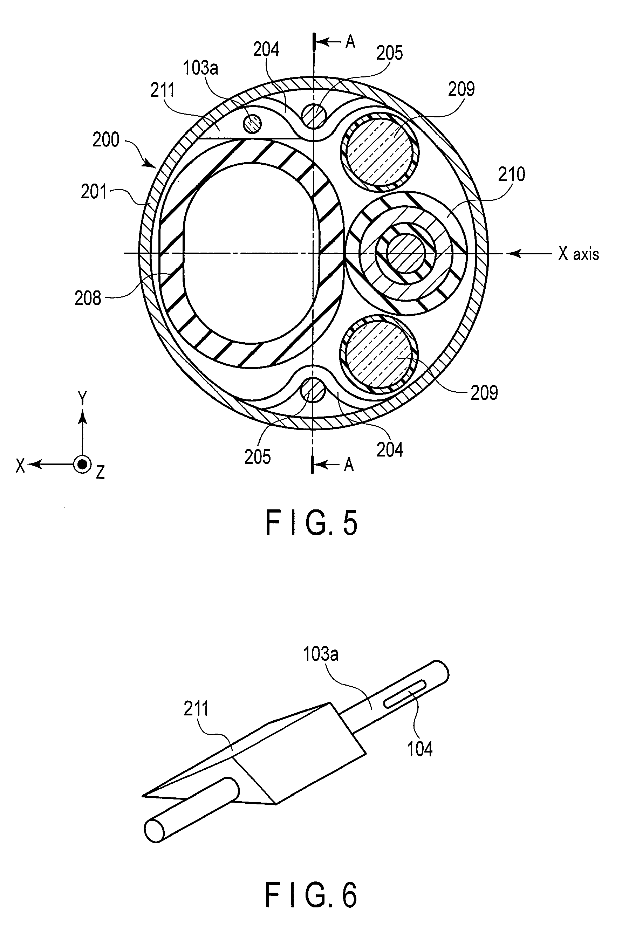

A description will now be given, with reference to FIGS. 4 and 5, of the internal structure of the distal insertion tube 154 of the endoscope 151 in which the curved shape sensor 101 of the present embodiment is incorporated. FIG. 4 is an axial-direction section of the distal insertion tube 154, taken along line A-A of FIG. 5. FIG. 5 is a radial-direction section of the distal insertion tube 154, taken along line B-B of FIG. 4.

The distal insertion tube 154 includes a tubular member 200 that has flexibility so as to be curved at least in one direction, and incorporation members located inside the tubular member 200.

The tubular member 200 includes a plurality of ring members 201 coupled to one another. The adjacent two ring members 201 are coupled, by means of a rivet 202, rotatably about the X axis shown in FIGS. 4 and 5 in the range of an operation curve section 220, and are coupled rotatably about the X-axis or Y-axis shown in FIGS. 4 and 5 in the range of a free curve section 221. A cap member 207 is rotatably coupled to the distal end of the distal insertion tube 154 by means of a ring member 201 and a rivet 202, and incorporation members, to be described later, are fixed and held within the cap member 207.

Each ring member 201 is provided with a guide 204, which is formed by press working and is integral with the ring member 201. The guide 204 has a holding hole through which an operating wire 205 can be inserted. The operating wire 205 is inserted through each guide 204 and is attached to the cap member 207.

In addition, the operating wire 205 is connected to the dial 162 of the proximal operation section 155 shown in FIG. 3. By rotating the dial 162, the operator can curve the distal insertion tube 154 in the upward or downward direction in the range of the operation curve section or in any direction desired in the range of the free curve section 221 to have a desirable curvature.

A CH tube 208 is arranged inside the tubular member 200 as an incorporation member. A treatment tool or the like can be inserted through the CH tube 208, and the distal end of the CH tube 208 is fixed and held by the cap member 207. As shown in FIG. 5, LG fibers 209 for illumination and a sensor cable 210 related to an image sensor for imaging are arranged inside the tubular member 200 as other incorporation members. Like the CH tube 208, the LG fibers 209 and the sensor cable 210 have their distal ends fixed and held by the cap member 207.

As shown in FIG. 5, the detection optical fiber 103a of the curved shape sensor 101 extends through the space defined by the ring members 201, the guides 204, and the CH tube 208, and the distal end of the detection optical fiber 103a is fixed and held by the cap member 207. A rotation suppressing member 211 is fixed to the detection optical fiber 103a of the curved shape sensor 101. In a cross section perpendicular to the axis of the detection optical fiber 103a, the rotation suppressing member 211 has a shape that can be received in the space defined by the ring members 201, guides 204, and CH tube 208 . The rotation suppressing member 211 is located within the space defined by the ring members 201, guides 204, and CH tube 208 with being in contact with the ring members 201, guides 204, and CH tube 208 or being pushed by them. With this structure, the rotation suppressing member 211 is not rotatable about the axis of the detection optical fiber 103a but is slidable in the axial direction of the detection optical fiber 103a. In this manner, the rotation suppressing member 211 suppresses rotation of the detection optical fiber 103a.

As shown in FIG. 4, the rotation suppressing member 211 is located near the detection part 104 with respect to the axial direction of the detection optical fiber 103a, but is away from the detection part 104 in consideration of the curved shape (curvature) of the distal insertion tube 154 so as not to affect the curvature of the detection part 104.

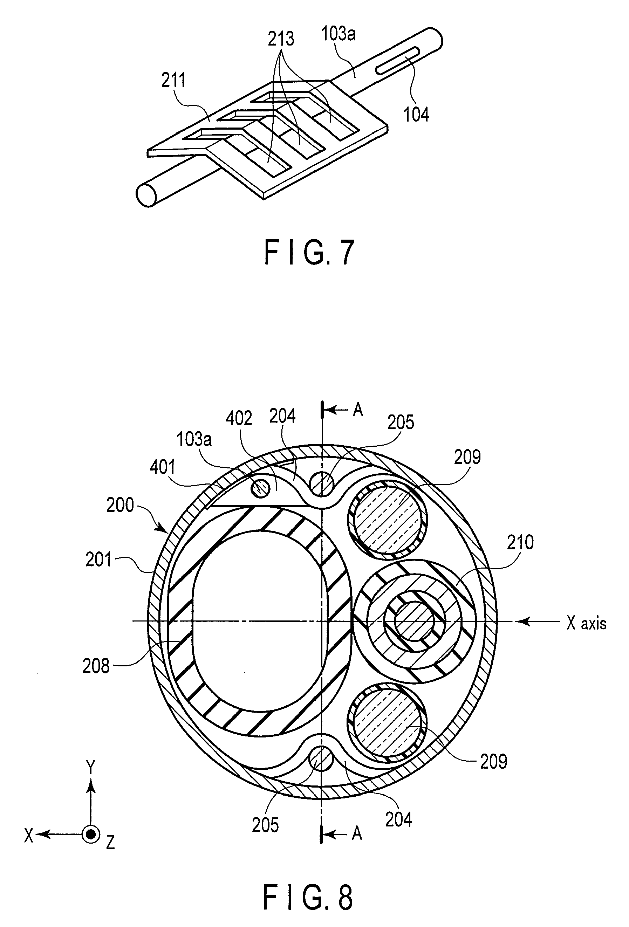

As shown in FIG. 6, the rotation suppressing member 211 may be constituted by a substantially triangle pole formed of elastic elastomer. Alternatively, as shown in FIG. 7, the rotation suppressing member 211 may be formed as a spring plate member that is bent to form an inverted "V", as viewed in the axial direction of the detection optical fiber 103a, and that have slits 213 extending in the X-axis direction so that the rotation suppressing member 211 has flexibility so as to be curved about the X-axis.

The detection optical fiber 103a is provided with at least one detection part 104 for detecting the curvature and curved direction. The detection part 104 is located at such a position as enables accurate detection of the curved shape of the distal insertion tube 154.

The detection optical fiber 103a is applied with a predetermined tensile force by a tension mechanism, not shown, provided within the proximal operation section 155.

In the present embodiment, the rotation suppressing mechanism 211 is pressed against the guide 204 by the CH tube 208. In place of this structure, the rotation suppressing mechanism 211 may be pressed by another incorporation member, such as the LG fiber 209 or the sensor cable 210. In addition, the rotation suppressing member 211 may be configured to be pressed against a guide.

In the present embodiment, the detection optical fiber 103a is fixed to and held by the cap member 207, and the rotation suppressing member 211 is attached to and held by the detection optical fiber 103a. In place of this structure, the detection optical fiber 103a may be attached to and held by another incorporation member, such as the CH tube 208. Furthermore, the rotation suppressing member 211 may be attached to and held by an incorporation member to which the detection optical fiber 103a is attached.

The detection optical fiber 103a does not have to be only one in number. Another detection optical fiber may be located in the space defined by the guide 204, the CH tube 208, and the ring member 201, depicted as being lower in, for example, FIG. 5.

### (Operation)

When the operator operates the dial 162 of the proximal operation section 155 shown in FIG. 3, the distal insertion tube 154 is rotated, with the X-axis shown in FIG. 5 (rivet 202) as a center of rotation, and is made to have a desired curvature. Since a tensile stress is generated on the outward side of the curve (the "plus" side of the Y-axis) and a compressive stress is generated on the inward side of the curve (the "minus" side of the Y-axis), the detection optical fiber 103a and the upper LG fiber 209, which are located on the outward side of the curve, are pulled toward the distal end, while the lower LG fiber 209, which is located on the inward side of the curve, is pushed toward the proximal end.

The detection optical fiber 103a may be moved in the axial direction by the forces generated in the incorporation members. At this time, the rotation suppressing member 211 is pressed against the guide 204, because of the elasticity and displacement of the CH tube 208 adjacent to the rotation suppressing member 211 and the elasticity and displacement of the sensor cable 210 and LG fiber 209 adjacent to the CH tube 208. As a result, the rotation suppressing member 211 suppresses a rotation or twist of the detection optical fiber 103a about the Z-axis and is simultaneously slidable in the axial direction of the detection optical fiber 103a.

### (Advantage)

Even if the incorporation members vary when the distal insertion tube 154 is curved, the detection optical fiber 103a of the curved shape sensor 101 can be curved without being twisted. In addition, the detection part 104 provided for the detection optical fiber 103a is hard to vary in direction. It is therefore possible to provide a high-precision curved shape sensor capable of detecting a curved shape of an endoscope with higher accuracy.

### <Second Embodiment>

### (Structure)

A second embodiment will be described with reference to FIG. 8. With respect to structures similar to those of the first embodiment, a description of them will be omitted.

The second embodiment differs from the first embodiment in that a sliding sheet 401 is interposed between a ring member 201 and a rotation suppressing member 402. Therefore, the rotation suppressing member 402 is in contact with the ring member 201 through the sliding sheet 401. In the longitudinal direction, the sliding sheet 401 is provided at least in the range of the ring member assembly portion 223 shown in FIG. 4. The sliding sheet 401 has a width greater than that of the rotation suppressing member 402.

The sliding sheet 401 has flexibility so as to be curved to have a curvature equal to or greater than that the distal insertion tube 154. The sliding sheet 401 reduces the friction resistance between the ring member 201 and the rotation suppressing member can be remarkably reduced, compared with it is not provided.

### (Operation)

When the distal insertion tube 154 is curved, the rotation suppressing member 402 slides in the axial direction of the detection optical fiber 103a, as in the first embodiment. Since the sliding sheet 401 is interposed between the rotation suppressing member 402 and the ring member 201 in the second embodiment, the edge of the rotation suppressing member 402 does not interfere with the ring member 201, and the rotation suppressing member 402 can slide without getting caught.

### (Advantage)

As compared with the first embodiment, the second embodiment can provide a high-precision curved shape sensor that is very reliable.

### <Third Embodiment>

### (Structure)

A third embodiment will be described with reference to FIG. 4 and FIGS. 9-11. With respect to structures similar to those of the first and second embodiments, a description of them will be omitted.

A tape member 301 formed of flexible resin such as polyimide is adhered to the detection optical fiber 103a of a curved shape sensor 101 by use of an adhesive 302 . The tape member 301 is employed as a member having a similar function to that of the rotation suppressing member 211 of the first embodiment, and the flexibility is maintained after the adhesion.

The tape member 301 is arranged within the distal insertion tube 154 so as to be in contact with the guide 204 and the CH tube 208. With this structure, the rotation suppressing member 301 is mechanically held so that it is not rotatable about the axis of the detection optical fiber 103a but it is slidable in the axial direction of the detection optical fiber 103a.

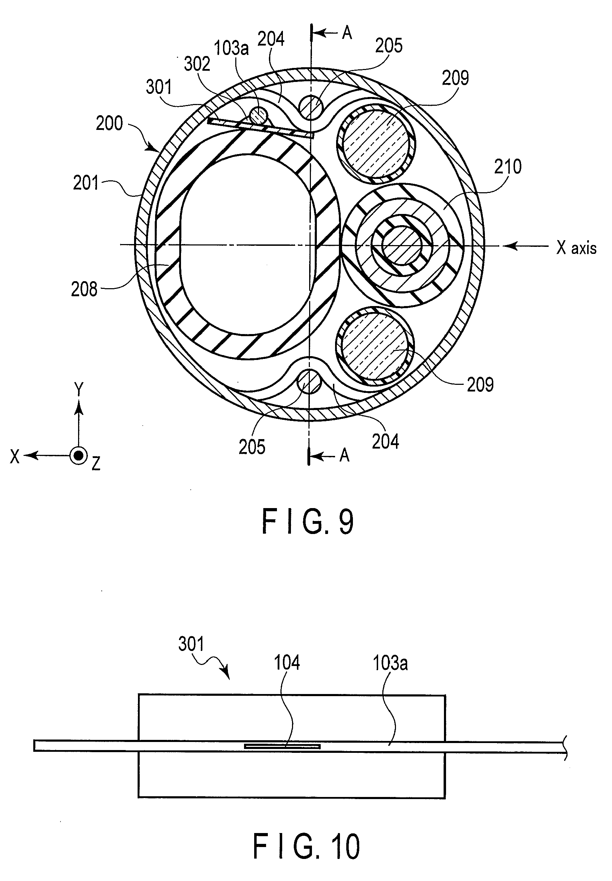

The tape member 301 has a rectangular shape that is elongated in the axial direction of the detection optical fiber 103a, as shown in FIG. 10. With this structure, the tape member 301 is easily curved about an axis extending in the width direction, but is hardly curved about an axis extending in the thickness direction. That is to say, the tape member 301 has flexibility so as to be curved in one direction. In this case, the tape member 301 is located so that its curved direction corresponds to a curved direction of the distal insertion tube 154. In other words, the tape member 301 is located with the thickness direction thereof generally corresponding to the Y-axis, so as to be easily curved about the X-axis.

The tape member 301 may have notches arrayed along sides extending in the axial direction of the detection optical fiber 103a, as shown in FIG. 11. In this case, the tape member 301 has flexibility so as to be curved in any direction desired. Therefore, the tape member 301 may be located within the distal insertion tube 154 with no need to consider the curved direction of the distal insertion tube 154.

In addition, since the tape member 301 has flexibility so as to be curved in any direction, it may be located in a free curve section 221 that can be curved in any direction.

In FIG. 11, the notches are formed throughout the whole length of the tape member 301. In place of this structure, notches may be cut only in a portion of the tape member 301. In this case, the portion of the tape member 301 in which no notch 303 is cut is located in the operation curve section 220, while the portion of the tape member in which the notches 303 are cut is located in the free curve section 221.

In addition, the tape member 301 need not be a rectangular flat tape; it may have a section having a cross-like figure, for example. In this case, in order to provide flexibility, notches have to be cut along sides extending in the axial direction of the detection optical fiber 103a.

### (Operation)

As in the first embodiment, the tape member 301 is in contact with the guide 204 and the CH tube 208. When the distal insertion tube 154 is curved, the tape member 301 suppresses a rotation or twist of the detection optical fiber 103a about the Z-axis and is simultaneously slidable in the axial direction of the detection optical fiber 103a.

Since the tape member 301 is continuous, it is more advantageous than the rotation suppressing member 211 of the first embodiment in that it does not interfere (get caught) with the edge of the ring member 201 during the axial-direction sliding when the distal insertion tube 154 is curved.

### (Advantage)

As compared with the first embodiment, the third embodiment can provide a high-precision curved shape sensor that is very reliable.

### <Fourth Embodiment>

### (Structure)

A fourth embodiment will be described with reference to FIG. 13. With respect to structures similar to those of the first to third embodiments, a description of them will be omitted.

A detection optical fiber 103a is adhered to a tape member 501 having stretchability equal to or greater than that of a distal insertion tube 154 by means of an adhesive (not shown) or the like, with respect to the lengthwise direction only. In other words, the tape member 501 is stretchable in the axial direction of the detection optical fiber 103a. With this structure, the tape member 501 has flexibility so as to be curved in any direction desired.

As in the third embodiment, the tape member 501 is arranged within the distal insertion tube 154 such that it is in contact with the guide 204 and the CH tube 208. With this structure, the tape member 501 is mechanically held so that it is not rotatable about the axis of the detection optical fiber 103a but it is slidable in the axial direction of the detection optical fiber 103a.

### (Operation)

As in the third embodiment, when the distal insertion tube 154 is curved, the tape member 501 suppresses a rotation or twist of the detection optical fiber 103a about the Z-axis and is simultaneously slidable in the axial direction of the detection optical fiber 103a. Since the tape member 501 is applied with a tensile stress and a compressive stress, but it is stretchable in the longitudinal direction, the detection optical fiber 103a can be curved in accordance with the curve of the distal insertion tube 154.

As in the third embodiment, the tape member 501 does not interfere (get caught) with the edge of the ring member 201.

### (Advantage)

As compared with the first embodiment, the fourth embodiment can provide a high-precision curved shape sensor that is very reliable.

While certain embodiments have been described, the present invention is not limited to those embodiments and may be modified or altered without departing from the spirit of the invention. The modification or alteration mentioned herein includes a proper combination of the aforesaid embodiments.

## Claims

1. A curved shape sensor for detecting a curved shape of a measured object, comprising:
a light source to emit detection light;
an optical fiber to guide the detection light;
a detection part provided at a portion of the optical fiber;
a rotation suppressing member fixed to the optical fiber; and
a light detection unit to detect the detection light traveling through the optical fiber,
the measured object including a tubular member that has flexibility so as to be curved at least in one direction, and an incorporation member located inside the tubular member,
the detection part changing characteristics of the detection light passing therethrough in accordance with a change in a curvature of the optical fiber, and
the rotation suppressing member being located within a space defined by the tubular member and the incorporation member with being in contact with the tubular member and the incorporation member, so as to suppress rotation of the optical fiber.

2. The curved shape sensor according to claim 1, wherein the tubular member is a distal insertion tube of an endoscope.

3. The curved shape sensor according to claim 2, wherein in a cross section perpendicular to the axis of the detection optical fiber 103a, the rotation suppressing member 211 has a shape that can be received in a space defined by the tubular member and the incorporation member.

4. The curved shape sensor according to claim 3, wherein the rotation suppressing member is located near the detection part in an axial direction of the optical fiber.

5. The curved shape sensor according to claim 3, wherein the rotation suppressing member is located at a position corresponding to the detection part in an axial direction of the optical fiber.

6. The curved shape sensor according to claim 5, wherein the rotation suppressing member can be curved to have a curvature equal to or greater than that of the optical fiber.

7. The curved shape sensor according to claim 6, wherein the rotation suppressing member is formed of an elastomer.

8. The curved shape sensor according to claim 6, wherein the rotation suppressing member is a metal plate member that has flexibility so as to be curved.

9. The curved shape sensor according to claim 3, further comprising a sliding member interposed between the tubular member and the rotation suppressing member, wherein the rotation suppressing member is in contact with the tubular member through the sliding member, and the sliding member reduces friction resistance between the tubular member and the rotation suppressing member, compared with the case where it is not provided.

10. The curved shape sensor according to claim 3, wherein the rotation suppressing member is a tape member that has flexibility so as to be curved at least in one direction.

11. The curved shape sensor according to claim 3, wherein the tape member is located so that its curved direction corresponds to a curved direction of the tubular member.

12. The curved shape sensor according to claim 10, wherein the tubular member has flexibility so as to be curved in any direction, and the tape member includes notches arrayed along an axis of the optical fiber, thereby permitting the tubular member to have flexibility in any direction.

13. The curved shape sensor according to claim 10, wherein the tubular member has flexibility so as to be curved in any direction, and the tape member is stretchable in an axial direction of the optical fiber, thereby permitting the tubular member to have flexibility in any direction.
